Europäisches Patentamt

European Patent Office

Office européen des brevets

(11) Veröffentlichungsnummer: **0 269 834 B1**

(12) **EUROPÄISCHE PATENTSCHRIFT**

(45) Veröffentlichungstag der Patentschrift: **11.09.91**

(51) Int. Cl.5: **G01N 21/78, G01N 33/18**

(21) Anmeldenummer: **87115540.4**

(22) Anmeldetag: **23.10.87**

(54) **Verfahren und Apparatur zur kontinuierlichen kolorimetrischen Bestimmung der Cyanidkonzentration wässriger Lösungen.**

(30) Priorität: **03.12.86 DE 3641251**

(43) Veröffentlichungstag der Anmeldung: **08.06.88 Patentblatt 88/23**

(45) Bekanntmachung des Hinweises auf die Patenterteilung: **11.09.91 Patentblatt 91/37**

(84) Benannte Vertragsstaaten: **AT BE CH DE ES FR GB IT LI LU NL SE**

(56) Entgegenhaltungen: **US-A- 3 850 761**

**ANALYTICAL CHEMISTRY, Band 24, Nr. 9, September 1952, Seiten 1440-1444; F.B. FISHER et al.: "Colorimetric determination of cyanide in stack gas and waste water"**

**LEHR- UND HANDBUCH DER ABWASSERTECHNIK, Band VII, "Industrieabwässer mit anorganischen Inhaltsstoffen", 1985, 3. Auflage, Seiten 206-216,261, W. Ernst & Sohn Verlag, Berlin, DE; L. HARTINGER: "Metallverarbeitende Industrie"**

**JOURNAL OF FOOD SCIENCE AND AGRICUL-**

**TURE, Band 31, 1980, Seiten 15-22,, Society of Chemical Industry; H.J. WILLIAMS et al.: "Estimation of cyanide with alkaline picrate"**

(73) Patentinhaber: **Degussa Aktiengesellschaft Weissfrauenstrasse 9 W-6000 Frankfurt am Main 1(DE)**

(72) Erfinder: **Bilger, Edgar, Dr. Sackgasse 4 W-6456 Langenselbold(DE)** Erfinder: **Wolf, Hubert Am Nussberg 19 W-6451 Hammersbach(DE)**

## Beschreibung

Die Erfindung betrifft ein Verfahren zur kontinuierlichen kolorimetrischen Bestimmung der Cyanidkonzentration wäßriger Lösungen, insbesondere Abwasser, wobei die Farbreaktion auf der Umsetzung von Cyanidionen mit Pikrinsäure im alkalischen Milieu beruht, aus Cyanokomplexen in Gegenwart von Chelatkomplexbildnern freisetzbares Cyanid miterfaßt wird und Cyanidkonzentrationen im Bereich von etwa 0,001 bis 5 mg CN/l spektralphotometrisch bestimmt werden. Das Verfahren läßt sich einfach und zuverlässig durchführen und gestattet es auch, Cyanidkonzentrationen im Konzentrationsbereich unter 0,2 mg CN/l ohne Extraktionsschritt zu erfassen.

Freie Cyanide und Cyanokomplexe enthaltende wäßrige Lösungen, insbesondere Abwässer, fallen in verschiedenen Industriezweigen in zum Teil sehr großen Mengen an, so zum Beispiel bei Prozessen zur Härtung und Vergütung von Metallen, bei der Erzaufbereitung durch Laugung und selektive Flotation, bei der Gichtgaswäsche von Hochofenprozessen, in der Galvanotechnik und in der chemischen Industrie. Auf Grund der hohen Toxizität verbietet es sich, solche Abwässer in Gewässer gelangen zu lassen, vielmehr müssen diese Abwässer entgiftet werden. Um die vom Gesetzgeber vorgeschriebenen oder empfohlenen Cyanid-Grenzwerte für das Einleiten von Abwässer in das Kanalnetz bzw. in freie Gewässer zu erreichen - im allgemeinen 0,1 bis 1,0 ppm - stehen verschiedene Verfahren zur Verfügung. Neben der lange bekannten Entgiftung mit Hypochlorit setzen sich zunehmend oxidative Verfahren mit dem umweltfreundlichen Wasserstoffperoxid durch; ferner finden auch andere Perverbindungen und andere Oxidationschemikalien zur Cyanidentgiftung Anwendung.

Unabhängig vom jeweils angewandten Verfahren erfordert jede Cyanidentgiftung ein auf sie abgestimmtes Analysenverfahren. Insbesondere ist dabei zu berücksichtigen, ob und in welchem Ausmaß das infrage stehende Analysenverfahren durch anwesende andere Stoffe, beispielsweise bestimmte Ionen, aus der Entgiftung stammende Reaktionsprodukte oder überschüssiges Entgiftungsreagenz gestört wird. Dem Fachmann stehen beispielsweise argentometrische, elektrochemische und kolorimetrische Analysenverfahren für die diskontinuierliche und zum Teil kontinuierliche Bestimmung der Cyanidkonzentration zur Verfügung, diese Verfahren lassen sich aber, wie schon dargelegt, wegen möglicher Störungen und wegen ihres unterschiedlichen Meßbereichs nicht allgemein anwenden.

An ein Verfahren zur kontinuierlichen Bestimmung der Cyanidkonzentration von z.B. Abwässern werden zunehmend höhere Anforderungen gestellt: Es muß eine zuverlässige und fortlaufende Erfassung der Cyanidkonzentration unter ständig wechselnden Betriebsbedingungen ermöglichen, die Erfassung auch sehr geringer Konzentrationen, um oder unter 0,1 mg CN/l, im Hinblick auf die steigenden Auflagen der Genehmigungsbehörden gewährleisten und auch unter harten Betriebsbedingungen wenig störanfällig und von chemisch-analytisch wenig geschultem Personal einfach und mit geringem Wartungsaufwand durchgeführt werden können; daneben sollen insbesondere die im Abwasser anwesenden anderen Stoffe, beispielsweise Cyanationen oder Phenole, und das Entgiftungsmittel, beispielsweise Wasserstoffperoxid, nicht stören.

Bekannt ist ein kontinuierliches elektrochemisches Verfahren zur Bestimmung der Cyanidkonzentration mittels potentiostatischer Anordnungen, siehe DECHEMA Monographie Nr. 75 (1974) Seiten 295 - 309. Dieses Verfahren erlaubt zwar die Messung geringer Cyanidkonzentrationen, ist in der Praxis jedoch sehr begrenzt einsatzfähig, da mit die wichtigste Voraussetzung, nämlich die Abwesenheit stark reduzierender und oxidierender Stoffe, sehr oft nicht gegeben ist. Beispielsweise stört Wasserstoffperoxid ; die Ausfällung des stark störenden Sulfids als Bleisulfid konnte in der Praxis nicht befriedigen.

Die bekannte Barbitursäure-Pyridin-Methode zur kolorimetrischen Bestimmung der Cyanidkonzentration beruht auf der Bildung eines Polymethinfarbstoffes, siehe E. Asmus und H. Garschagen in Zeitschrift für Analytische Chemie, Band 138, Seite 414 - 422 (1953).

Dieses Verfahren wurde auch schon für die kontinuierliche kolorimetrische Cyanidbestimmung verwendet, es weist jedoch einige schwerwiegende Nachteile auf, welche die Anwendbarkeit einschränken. So stören zum Beispiel Reduktionsmittel, Rhodanid-, Sulfit-, Sulfid-, Cyanat- und Hexacyanoferrationen sowie Wasserstoffperoxid. Bei der diskontinuierlichen Bestimmung kann man diese Störungen vermeiden, indem man aus den Cyaniden die Blausäure freisetzt, diese in eine Vorlage mit Natronlauge überführt und dort bestimmt; es ist klar, daß diese Vorgehensweise bei einer kontinuierlichen Bestimmung nicht anwendbar ist. Weitere Nachteile sind die zeitlich sehr begrenzte Alterungsbeständigkeit der für die Farbbildung benötigten Chloramin T- und Pyridin-Barbitursäure-Reagenzlösung und die Tatsache, daß Cyanide bestimmter Metalle, wie z.B. Nickel, Kupfer, Silber und Gold nicht oder nur sehr beschränkt erfaßbar sind.

Sehr eingehend wurde die lange bekannte Isopurpurat-Reaktion der Pikrinsäure zum qualitativen und quantitativen Nachweis von Cyanidionen untersucht, siehe z.B. F.B. Fisher und J.S. Brown in Analytical Chemistry Vol. 24 (1952) No. 9, Seiten 1440 - 1444. Zur diskontinuierlichen kolorimetrischen Cyanidbestimmung kann das von D.J. Barkley und J. C. Ingles verbesserte Verfahren, durch welches es möglich wurde, neben freiem Cyanid auch aus Cyanokomplexen freisetzbares Cyanid zu erfassen, herangezogen werden - siehe Research Report R 221 Department of Energy, Mines and Resources, Mines Branch, Ottawa, Febr. 1970. Die Bildung des Cyanid-Pikrinsäure-Farbkomplexes wird im Gegensatz zur Barbitursäure-Pyridin-Methode durch Stoffe, wie Wasserstoffperoxid, Phenole, Cyanat-, Rhodanid-, Thiosulfat-und Sulfitionen im allgemeinen nicht oder erst in Gegenwart sehr hoher Konzentrationen gestört.

Ein zunächst unerkanntes Problem der diskontinuierlichen Cyanid-Pikrinsäure-Methode zeigte sich aber darin, daß Blausäure-Ausgasungsverluste nicht vollständig zu vermeiden sind und damit zu niedrige Cyanid-Konzentrationswerte gefunden werden: Die Abweichungen vom Sollwert sind bei der Bestimmung freien Cyanids im allgemeinen größer als im Falle des aus Cyanokomplexen freisetzbaren Cyanids.

Barkley und Ingles geben keinerlei Hinweise, das diskontinuierliche Verfahren kontinuierlich durchzuführen. Dies lag auch nicht nahe,weil zur Bestimmung von Konzentrationen unter 0,2 ppm der zunächst in wäßriger Phase gebildete Farbkomplex vor der Extinktionsmessung in einem weiteren Verfahrensschritt mit Chloroform in Gegenwart eines quaternären Ammoniumsalzes extrahiert werden mußte, und der Zeitaufwand für eine einzelne Bestimmung etwa eine Stunde betrug. Die Notwendigkeit, ein organisches Lösungsmittel verwenden zu müssen, sowie der erhebliche zeitliche und apparative Aufwand standen einer einfach und mit geringem Wartungsaufwand der Apparatur durchführbaren kontinuierlichen Bestimmung entgegen.

Aufgabe der vorliegenden Erfindung ist es, ein auf der bekannten kolorimetrischen Cyanid-Pikrinsäure-Farbreaktion beruhendes verbessertes Verfahren zu schaffen, das es in einfacher und zuverlässiger Weise gestattet, die Cyanidkonzentration wäßriger Lösungen im Bereich von etwa 0,001 bis 5 mg Cyanid pro Liter kontinuierlich zu bestimmen, die Fehlermöglichkeit durch die HCN-Ausgasung zu vermeiden und auf die Verwendung eines organischen Lösungsmittels und den Extraktionsschritt zu verzichten.

Gelöst wird diese Aufgabe durch ein Verfahren zur kolorimetrischen Bestimmung der Cyanidkonzentration wäßriger Lösungen durch Umsetzung des freien und in Gegenwart von Chelatkomplexbildnern aus Cyanokomplexen freisetzbaren Cyanids mit Pikrinsäure in wäßrigem alkalischen Milieu bei gegenüber Raumtemperatur erhöhter Temperatur und spektralphotometrische Messung der Extinktion des gebildeten roten Cyanid-Pikrat-Farbkomplexes bei etwa 520 nm, das dadurch gekennzeichnet ist, daß man Cyanidkonzentrationen im Bereich von etwa 0,001 bis 5 mg Cyanid pro Liter Probelösung erfaßt, indem man die Bestimmung kontinuierlich durchführt, wobei man in einem gasdicht geschlossenen System einen kontinuierlichen Strom der freies und/oder freisetzbares Cyanid enthaltenden Probelösung mit den zur Farbbildung, Cyanidfreisetzung und pH-Einstellung erforderlichen Chemikalien unter Einhaltung konstanter Konzentrationen in Kontakt bringt, das Lösungsgemisch zur Bildung des Farbkomplexes in einem rohrförmigen Durchflußreaktor 1 bis 60 Minuten auf 50 bis 120 °C erwärmt, anschließend die Lösung auf die für die Extinktionsmessung vorgesehene Temperatur bringt, den für die Durchflußmeßküvette zulässigen Druck einstellt und dann in an sich bekannter Weise kontinuierlich die Extinktion der durch die Durchflußmeßküvette eines Spektralphotometers strömenden Lösung unter Berücksichtigung der Extinktion einer cyanidfreien Blindprobe mißt.

Die weiteren Ansprüche betreffen bevorzugte Ausführungsformen des Verfahrens sowie eine besonders geeignete Apparatur zu seiner Durchführung.

Es wurde überraschend gefunden, daß man die Cyanidkonzentration von freiem Cyanid und/oder in Gegenwart von Chelatkomplexbildnern aus Cyanokomplexen freisetzbarem Cyanid enthaltenden wäßrigen Lösungen sicher reproduzierbar und sehr genau bestimmen kann, wenn man in einem gasdicht geschlossenen System, wie es beispielsweise in der baschriebenen Apparatur leicht zu realisieren ist, sowohl die Probelösung mit der alkalischen Pikrinsäure-Reagenzlösung miteinander in Kontakt bringt als auch die Umsetzung zum Cyanid-Pikrat-Farbkomplex vornimmt. Unter einem gasdicht geschlossenen System wird ein solches verstanden, das nur eine Flüssigkeitsphase beinhaltet, das heißt, im System praktisch keine Gasphase vorhanden ist. Unter dieser Voraussetzung erhält man bei gleicher Cyanidkonzentration bei der spektralphotometrischen Extinktionsmessung gleiche Extinktionswerte, unabhängig davon, ob die Probelösung freies Cyanid, z.B. Na CN, oder Cyanokomplexe, z.B. Na [Ag (CN)$_2$] oder Na$_2$ [Ni (CN)$_4$], oder ein Gemisch beider enthält - vergleiche nicht geschlossenes System gemäß Beispiel 1 mit dem gasdicht geschlossenen System gemäß Beispiel 2. Darüber hinaus liegen die in Abhängigkeit von der Konzentration aufgetragenen Extinktionswerte bei der Bestimmung im geschlossenen System auf einer Geraden.

Die durch das gasdicht geschlossene System überraschend erzielten vorteilhaften Wirkungen sind wesentliche Voraussetzungen für ein kontinuierliches Verfahren zur Bestimmung der Cyanidkonzentration wäßriger Lösungen mit gegebenenfalls rasch wechselnder Konzentration und hinsichtlich der Art der Cyanoverbindung wechselnder Zusammensetzung.

Es wurde darüber hinaus gefunden, daß man bei der kontinuierlichen Bestimmung der Cyanidkonzentration in einem gasdicht geschlossenen System nicht nur Cyanidkonzentrationen im Bereich zwischen 0,2 und wenigen mg CN/l direkt, das heißt, ohne einen zusätzlichen Extraktionsschritt oder anderweitige Konzentrierung der gefärbten Lösung durch Extinktionsmessung des in wäßriger Phase gebildeten Cyanid-Pikrat-Farbkomplexes sicher erfassen kann, sondern auch solche, welche im Bereich von etwa 0,001 bis 0,2 mg Cyanid pro Liter liegen. Der gesamte durch das erfindungsgemäße Verfahren erfaßbare Meßbereich beträgt etwa 0,001 bis 5 mg Cyanid pro Liter wäßriger Lösung, besonders gut erfaßbar sind 0,01 bis 3 mg/l. Der erreichbare untere Grenzwert hängt gegebenenfalls vom verwendeten Spektralphotometer ab; die Erfassung der sehr geringen Konzentrationen, insbesondere solcher unter 0,01 mg CN/l, erfordert im allgemeinen die Verwendung eines Gerätes mit sehr gutem Meßverstärker und Trübungskompensationseinrichtung. Im allgemeinen ist der Fehler im Konzentrationsbereich unter 0,01 mg CN/l größer als im Bereich darüber.

Die Bestimmung von Cyanidkonzentrationen über 5 mg/l ist zwar nach dem erfindungsgemäßen Verfahren möglich, um genaue Meßwerte zu erhalten, empfiehlt es sich jedoch, die Probelösung vor, während oder nach Eindosieren in die erfindungsgemäße Apparatur so zu verdünnen, daß im Lösungsgemisch eine Cyanidkonzentration um 3 mg/l nicht überschritten wird.

Im allgemeinen wird man die zur Farbbildung, Cyanidfreisetzung und pH-Einstellung und gegebenenfalls Pufferung erforderlichen Chemikalien in Form einer oder mehrerer wäßriger Lösungen der zu bestimmenden Lösung zusetzen. Besonders bevorzugt verwendet man eine alle erforderlichen Chemikalien enthaltende alkalische Pikrinsäure-Reagenzlösung. Der Fachmann wird durch einfache Versuche zuvor prüfen, ob die ausgewählten Chelatkomplexbildner, Basen bzw. Pufferkombinationen mit der Pikrinsäure verträglich sind und eine lagerstabile Lösung geben.

Die Dosierung der Probelösung und Reagenzlösung bzw. -lösungen erfolgt vorteilhafterweise mittels fein regulierbarer Dosierpumpen in eine Mischzone, welche zum Beispiel als einfaches oder mit statischen Mischvorrichtungen versehenes Rohrstück ausgebildet sein kann. Von der Mischzone gelangt das Lösegemisch zur Bildung des Farbkomplexes in einen heizbaren rohrförmigen Durchflußreaktor. Dieser rohrförmige Durchflußreaktor hat bevorzugt einen geringen Innendurchmesser und ist im allgemeinen spiralförmig in einem Thermoblock angeordnet. Zur Farbbildung pumpt man das Lösegemisch durch den Reaktor, wobei man dieses 1 bis 60 Minuten auf 50 ° bis 120 °C, bevorzugt 5 bis 20 Minuten auf 80 bis 110 °C, erwärmt. Die erforderliche Verweilzeit bei gegebener Temperatur hängt sowohl von der Zusammensetzung der Reagenzlösung als auch den in der Probelösung gegebenenfalls enthaltenen Cyanokomplexen, aus welchen Cyanid in Gegenwart von Chelatkomplexbildnern unterschiedlich rasch freigesetzt wird, ab; im unteren Temperaturbereich benötigt man daher eine längere Reaktionszeit als im oberen Temperaturbereich. Die Strömungsgeschwindigkeit im rohrförmigen Durchflußreaktor wird man so einstellen, daß eine nennenswerte Rückvermischung während der Verweilzeit im Reaktor nicht eintritt.

Nach dem erfindungsgemäßen Verfahren unter Verwendung des rohrförmigen Durchflußreaktors wird das Lösungsgemisch innerhalb sehr kurzer Zeit auf die gewünschte Reaktionstemperatur gebracht. Man kann die Farbbildung auch bei Temperaturen zwischen dem Siedepunkt von Wasser und 120 °C durchführen, wenn man dafür sorgt, daß durch eine bevorzugt nach dem Durchflußkühler angeordnete Vorrichtung, z.B. ein Drosselventil, der entsprechende Gegendruck aufgebaut und die Bildung von Gasblasen vermieden wird. Beide Merkmale sind dazu geeignet, die Reaktionszeit möglichst niedrig zu halten, was insbesondere von Bedeutung ist, wenn die kontinuierliche Cyanid-Konzentrationsbestimmung zur Steuerung einer kontinuierlichen Cyanidentgiftung oder zur Abwasserendkontrolle eingesetzt wird, oder wenn die Probelösung solche Cyanokomplexe enthält, z.B. $Ag(CN)_2^-$, aus welchen das Cyanid weniger leicht freisetzbar ist. Während eine quantitative Erfassung von in $Ag(CN)_2$ gebundenem Cyanid nach dem bekannten diskontinuierlichen Verfahren oft nicht oder nur nach sehr langer Reaktionszeit bei Siedetemperatur möglich war, gelingt dies nach dem erfindungsgemäßen Verfahren problemlos.

Unter dem erfindungsgemäß erfaßbaren Cyanid wird das in wäßriger Lösung dissoziiert vorliegende Cyanid verstanden. Bei dem aus Cyanokomplexen freisetzbaren Cyanid handelt es sich insbesondere um solches, welches aus Cyanokomplexen des Zinks, Cadmiums, Kupfers, Nickels und Silbers in Gegenwart von Chelatkomplexbildnern freigesetzt und damit der Farbbildung zugänglich gemacht wird. Cyanoaurate setzen unter den üblichen Reaktionsbedingungen durch Umkomplexierung mit DTPA Cyanid meist nur unvollständig frei. Cyanokomplexe des Kobalts und Eisens setzen dagegen Cyanid nicht oder nur in geringem Umfang frei.

Zur Umkomplexierung der Cyanokomplexe kommen Chelatkomplexbildner, insbesondere vier- und höherzählige Chelatkomplexbildner, zur Anwendung. Die Chelatbildner können beispielsweise als funktionelle Gruppen je Molekül entweder Hydroxyl- und Carboxylgruppen, mehrere Aminogruppen oder Amino- und Carboxylgruppen oder Amino- und Phosphonatgruppen enthalten. Einsatzbar sind z.B. Weinsäure, Diethylentriamin, Nitrilotriessigsäure; bevorzugt eingesetzt werden jedoch stärkere Chelatkomplexbildner, wie Ethylendiamin-tetraessigsäure (EDTA) oder Diethylentriamin-pentaessigsäure (DTPA) oder Ethylendiamin-tetrakis(methylenphosphonat)bzw. deren wasserlösliche Salze, beispielsweise Alkalisalze.

Die Farbbildung zum Cyanid-Pikrat-Farbkomplex ist hinsichtlich seiner Bildungsgeschwindigkeit und Farbintensität vom pH-Wert abhängig. Üblicherweise führt man die Umsetzung in wäßrigem alkalischen Milieu durch, wobei jedoch ein pH-Wert im Bereich von etwa 7,1-12 bevorzugt, ein solcher von etwa 9 ± 0,5 besonders bevorzugt ist.

Um sicher reproduzierbare Ergebnisse der Cyanidkonzentrationsbestimmung auch bei rasch wechselndem Cyanidgehalt und pH-Wert und unterschiedlicher Zusammensetzung der wäßrigen Probelösung zu erhalten, ist es vorteilhaft, die Umsetzung zum Farbkomplex in Gegenwart von einem oder mehreren den pH-Wert puffernden Säure-Basen-Paaren vorzunehmen. Bei der Auswahl und der Menge der Säure-Base-Paare wird der Fachmann davon auszugehen haben, daß er den gewünschten pH-Wert, ein auf den Anwendungsfall abgestimmtes breites Pufferniveau und genügende Pufferkapazität erhält.

Zur pH-Einstellung können insbesondere Alkalihydroxide und/oder Alkalicarbonate und/oder andere alkalisch wirkende Verbindungen verwendet werden. Als Säure-Base-Paare zur Pufferung eignen sich besonders aus Borax und Natriumhydroxid zugängliche Puffer; auch Chelatkomplexbildner, wie beispielsweise EDTA oder DTPA, wirken in Gegenwart ihrer Alkalisalze als puffernde Säure-Base-Paare.

Besonders vorteilhaft ist es, die Cyanide enthaltende Probelösung kontinuierlich mit einer solchen Menge einer Chelatkomplexbildner enthaltenden alkalisch gepufferten Pikrinsäure-Reagenzlösung zu versetzen, daß das Lösungsgemisch einen pH-Wert zwischen 8,5 und 9,5 aufweist, und je Liter etwa 0,001 bis 3 mg freies und/oder freisetzbares Cyanid enthält und wobei man je Liter 0,5 bis 3,0 g Pikrinsäure, 2 bis 20 g EDTA oder DTPA, 1 bis 10 g NaOH und 1 bis 10 g $Na_2B_4O_7$ verwendet.

Im allgemeinen kühlt man das den Durchflußreaktor verlassende Lösungsgemisch, zum Beispiel in einem Durchflußkühler, auf die Temperatur ab, bei welcher die Extinktionsmessung vorgenommen wird. Meist wird man das Lösungsgemisch auf 15 bis 30 °C abkühlen und bei dieser Temperatur, im allgemeinen nach Druckentlastung auf Normaldruck, durch die Durchflußküvette eines Spektralphotometers strömen lassen.

Zur Extinktionsmessung können Ein- oder Zweistrahlspektralphotometer, welche bevorzugt mit einer Vorrichtung zur Trübungskompensation, wodurch eine Störung durch Trübung hervorrufende Schwebstoffe weitgehend verhindert wird, ausgestattet sind, verwendet werden.

Den Ausgang des kontinuierlich arbeitenden Photometers verbindet man zur Aufzeichnung der Meßwerte vorteilhafterweise mit einem Linienschreiber.

Die Nullpunkteinstellung mittels einer cyanidfreien Blindprobe, die Aufstellung einer Eichkurve mittels Eichlösungen und die Messung der Extinktion der Probelösungen erfolgen in bekannter Weise. Zu Kontrollzwecken schaltet man in Intervallen von der Probelösung auf die Blindprobe oder gegebenenfalls auch Eichlösungen um und behandelt diese erfindungsgemäß. Die Schichtdicke der Durchflußküvette beträgt im allgemeinen 1 bis 8 cm. Man mißt bei einer Wellenlänge zwischen 510 und 530 mm, bevorzugt bei 520 mm.

Der technische Fortschritt des erfindungsgemäßen Verfahrens besteht darin, daß es nun erstmals möglich ist, die Cyanid-Pikrat-Farbreaktion zur kontinuierlichen Konzentrationsbestimmung zu nutzen und damit dieses Verfahren zur kontinuierlichen Kontrolle von Abwasserströmen oder zur Steuerung von Cyanid-Entgiftungsverfahren einzusetzen. Des weiteren können erfindungsgemäß auch solche geringen Cyanidkonzentrationen in einfacher Weise direkt bestimmt werden, wozu bei dem bekannten diskontinuierlichen Verfahren ein zusätzlicher Schritt, nämlich eine Extraktion, mit einem organischen Lösungsmittel erforderlich war. Wesentliche Vorteile gegenüber dem bekannten diskontinuierlichen Verfahren sind darüber hinaus die sicher reproduzierbare Erfaßbarkeit der Cyanidkonzentration, unabhängig von deren absoluter Höhe und Vorliegen als freies und/oder freisetzbares Cyanid, der geringe manuelle Aufwand zum Bedienen und Warten der Meßapparatur und der geringe Chemikalienaufwand - mit der bisher für eine einzige Bestimmung erforderlichen Menge lassen sich nun während einer bestimmten Betriebsdauer praktisch beliebig viele Meßdaten erfassen.

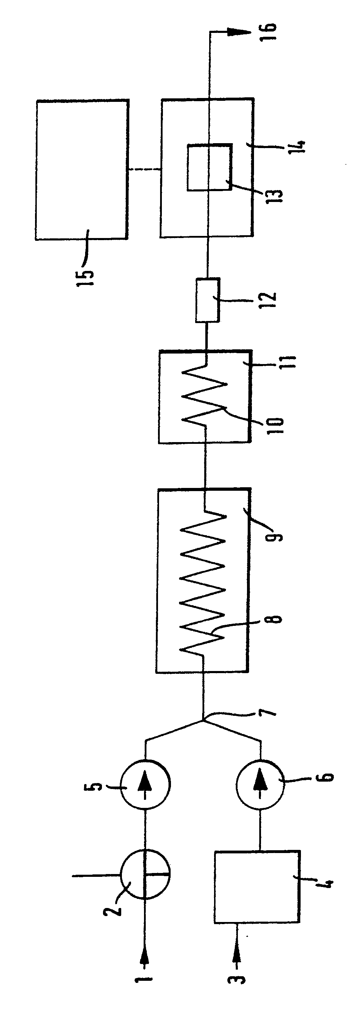

Die kontinuierliche Bestimmung der Cyanidkonzentration nach dem erfindungsgemäßen Verfahren läßt sich besonders vorteilhaft in einer Apparatur gemäß Figur durchführen, welche Vorrichtungen zum kontinuierlichen Dosieren (5) bzw.(6) der Cyanid enthaltenden Probelösung (1) bzw. einer oder mehrerer die Chemikalien zur Farbbildung, Cyanidfreisetzung und pH-Einstellung enthaltenden wäßrigen Lösungen (3), Vorrichtungen zum Zusammenführen und Mischen dieser Lösungen (7), einen mit Vorrichtungen zum Beheizen (9) auf 50 bis 120 °C versehenen rohrförmigen Durchflußreaktor (8), einen mit Vorrichtungen zum Kühlen (11) auf bis zu 10 °C der den Reaktor verlassenden Lösung versehenen rohrförmigen Durchflußkühler (10), daran anschließend Vorrichtungen zum Regulieren des Drucks (12) und ein Spektralphotometer (14), vorteilhaft verbunden mit einem Linienschreiber (15) zum kontinuierlichen Aufzeichnen der Extinktion, enthält, und die das Lösungsgemisch führenden Vorrichtungen zwischen den Dosierstellen (5) bzw. (6) und der Druckregulierung (12) ein gasdicht geschlossenes System bilden. In der Figur bedeuten (4) ein Vorratsgefäß für die alle Chemikalien enthaltende Pikrinsäure-Reagenzlösung, (9) den Ablauf und (13) die Durchflußküvette des Spektralphotometers (14), (2) einen Dreiwegehahn zur Umstellung von der Probelösung auf die Blind-/Eichlösung.

Wie schon erwähnt, ist es erfindungswesentlich, daß sich beim Zusammenführen der Lösungen und Umsetzung bei erhöhter Temperatur zum Farbkomplex praktisch keine Gasphase bildet, welche die Ergebnisse verfälschen könnte, und eine nennenswerte Rückvermischung innerhalb der Apparatur nicht stattfindet: dem wird bei der Ausgestaltung der Apparatur Rechnung getragen, wenn man die Vorrichtungen in der zuvor angegebenen Reihenfolge anordnet und die Lösungsgemisch führenden Teile druckfest ausführt -mindestens dem Wasserdampfdruck der maximalen Temperatur des Durchflußreaktors entsprechend.

Der rohrförmige Durchflußreaktor (8) mit geringem Innendurchmesser weist vorteilhafterweise ein Volumen von 10 bis 250 ml und einen Durchmesser von 1 bis 5 mm auf. Die Anordnung des Durchflußreaktors wird man nach dem Heizaggregat richten: vorteilhafterweise ordnet man den Durchflußreaktor (8) spiralförmig an, um ihn auf kleinem Raum, beispielsweise in einem Thermoblock (9), der ein Festbett- oder Flüssigbettthermoblock sein kann, unterzubringen.

Die Ausführung des kühlbaren Durchflußkühlers (10, 11) gleicht vorteilhafterweise derjenigen des Reaktors, wobei ein Volumen von 1 bis 25 ml den Anforderungen zum Kühlen im allgemeinen gerecht wird.

Zum Aufbau eines Drucks im Reaktionsteil der Apparatur und zum Entspannen des auf Extinktions-Meßtemperatur abgekühlten Lösungsgemischs dienen bekannte Druckregelungsgeräte (12), bevorzugt ist ein Drosselventil. An sich kann jedes empfindliche Ein- oder Zweistrahl-Spektralphotometer verwendet werden, vorteilhaft ist ein Spektralphotometer mit Trübungskompensation.

Mit der erfindungsgemäßen Apparatur kann man das Cyanidentgiftungsergebnis unter Umgehung der zeitaufwendigen Analyse auf "leicht freisetzbares Cyanid" nach DIN 38 405 D 13.2 kontinuierlich durchführen. Die Apparatur zeichnet sich durch einfachen Aufbau, einfache Bedienbarkeit, hohe Genauigkeit und gute Erfaßbarkeit im Bereich von Konzentrationen zwischen etwa 0,001 bis 5 mg Cyanid pro Liter aus und ist in vielen Bereichen der chemischen Industrie und Minentechnik einsetzbar.

Beispiel 1 (Stand der Technik)

Diskontinuierliche Bestimmung von Cyanid.

Zur Messung der Extinktion des aus NaCN stammenden freien Cyanids bzw. des aus $Na_2$ [Ni(CN)$_4$] in Gegenwart von Diethylentriamin-pentaessigsäure (DTPS) freisetzbaren Cyanids wurden jeweils aliquote Mengen mit 25, 50, 100, 150 und 200 µg Cyanid von zuvor hergestellten NaCN - bzw. $Na_2$ [Ni(CN)$_4$] -Standardlösungen entnommen, in 100 ml Erlenmeyerkolben überführt und auf gleiches Volumen von 70 ml gebracht. Hierzu sowie zu einer Blindprobe wurden jeweils 25 ml der gepufferten und DTPA enthaltenden alkalischen Pikrinsäure-Reagenzlösung zugefügt - die Reagenzlösung wurde durch Auflösen von 6 g Pikrinsäure, 40 g DTPA, 16 g NaOH in etwa 800 ml Wasser, Zugabe von 14 g $Na_2B_4O_7$ und 14 g $Na_2CO_3$ und Auffüllen auf 1000 ml hergestellt. Die mit der Reagenzlösung versetzten Proben wurden 30 Minuten auf dem siedenden Wasserbad erwärmt, auf 25 °C abgekühlt, in 100 ml-Meßkolben überführt und auf Volumen gebracht. In einem Einstrahl-Spektralphotometer wurde bei 520 mm in üblicher Weise unter Verwendung von Küvetten mit einer Schichtdicke von 1 cm die Extinktion im Vergleich zur Blindprobe gemessen. Wie die Ergebnisse der nachstehenden Tabelle zeigen, liegen die Extinktionswerte nicht auf einer Geraden, welche an sich, wie durch eine Verdünnungsreihe festgestellt wurde, in diesem Bereich Gültigkeit hat. Die Meßwerte der den Cyanokomplex enthaltenden Proben liegen über denen der die korrespondierende Menge freies Cyanid enthaltenden Proben.

| µg CN/Probe | Extinktion | |
|---|---|---|
| | NaCN | $Na_2 Ni(CN)_4$ |
| 25 | 0,053 | 0,055 |
| 50 | 0,104 | (0,127) |
| 100 | 0,205 | 0,210 |
| 150 | 0,294 | 0,314 |
| 200 | 0,385 | 0,404 |

Beispiel 2

Kontinuierliche Bestimmung der Cyanidkonzentration.

Die erfindungsgemäße Apparatur bestand aus einem Dosierbehälter für die Pikrinsäure-Reagenzlösung, gleiche Zusammensetzung wie in Beispiel 1, einem über ein Dreiwegemagnetventil umstellbaren Zulauf für die Probelösung bzw. Eichlösung, je einer Dosierpumpe für die Reagenz- bzw. Probelösung, einem in einem Festbettthermoblock eingegossenen, spiralförmig angeordneten, rohrförmigen Durchflußreaktor aus V4A-Stahl mit einem Volumen von 150 ml und einem Innendurchmesser von 5 mm, einem in gleicher Weise aufgebauten, in einen Kühlblock eingegossenen Durchflußkühler mit einem Volumen von 25 ml, einem Drosselventil und einem Einstrahl-Spektralphotometer mit Trübungskompensation, dessen Ausgang mit einem Linienschreiber verbunden war.

Es wurden NaCN- und Na [Ag(CN)2] enthaltende Probelösungen mit jeweils 1,82 mg, 0,73 mg, 0,36 mg und 0,07 mg freiem bzw. freisetzbarem Cyanid pro Liter Lösung hergestellt.

Zur Eichung wurden zunächst Wasser und Reagenzlösung im Verhältnis 3 : 1 mittels der Dosierpumpen zusammengeführt, in einem als Mischer dienenden Rohrstück gemischt und nach Passieren des auf 100 °C geheizten Thermoblocks, Abkühlen auf 25 °C und Entspannen dem Spektralphotometer zur Nullpunkteinstellung zugeführt; 8 cm-Küvette, Meß-Wellenlänge 520 nm.

Der Durchfluß des Lösungsgemischs betrug 1,2 l/Std., die Verweilzeit zwischen Dosieren und Messen 17 Minuten. Nach Umschalten des Dreiwegemagnetventils auf die zu bestimmende Lösung wurden die Probelösungen unter Beibehaltung der übrigen Bedingungen hintereinander, jeweils über eine Zeit von 5 Minuten, zudosiert. Die Meßwerte folgen aus der Tabelle und zeigen, daß freies $CN^-$ und aus $Ag(CN)_2^-$ freisetzbares Cyanid und Gemische beider in gleicher Weise erfaßt werden und alle Meßwerte mit nur geringer Streubreite auf einer Geraden liegen.

| mg CN⁻/l (freies bzw. freisetzbares Cyanid) | Extinktion | |
|---|---|---|
| | NaCN | Na[Ag(CN)₂] |
| 1,82 | 0,302 | 0,306 |
| 0,73 | 0,121 | 0,121 |
| 0,36 | 0,061 | 0,062 |
| 0,07 | 0,013 | 0,013 |
| 0,36  *) | 0,062 | |
| 0,07  *) | 0,012 | |

*) 1 : 1 Mischung der NaCN und Na[Ag(CN)₂] enthaltenden Lösungen gleicher Konzentration.

Beispiel 3

Diskontinuierliche Bestimmung - Einfluß der Pufferung der Reagenzlösung.

Gemäß Beispiel 1 wurden 80 μg Cyanid als NaCN bzw. Na₂[Ni(CN)₄] enthaltende Proben mit je 25 ml der nachstehenden Reagenzlösungen versetzt und nach Farbbildung die Extinktion des gebildeten Cyanid-Pikratkomplexes gemessen.

| Reagenzlösung | Extinktion | |
|---|---|---|
| | 80 μg CN⁻ als NaCN | 80 μg CN⁻ als Ni(CN)₄⁻⁻ |
| a)  *) | 0,122 | 0,148 |
| b)  *) | 0,163 | 0,169 |

*) a) = 6 g Pikrinsäure + 40 g DTPA + 16 g NaOH pro l Reagenzlösung

b) = wie a), jedoch zusätzlich 14 g Na₂B₄O₇.

Patentansprüche

1. Verfahren zur kolorimetrischen Bestimmung der Cyanidkonzentration wäßriger Lösungen durch Umsetzung des freien und in Gegenwart von Chelatkomplexbildnern aus Cyanokomplexen freisetzbaren Cyanids mit Pikrinsäure in wäßrigem alkalischen Milieu bei gegenüber Raumtemperatur erhöhter Temperatur und spektralphotometrische Messung der Extinktion des gebildeten roten Cyanid-Pikrat-Farbkomplexes bei etwa 520 nm, dadurch gekennzeichnet, daß man Cyanidkonzentrationen im Bereich von etwa 0,001 bis 5 mg Cyanid pro Liter Probelösung erfaßt, indem man die Bestimmung kontinuierlich durchführt, wobei man in einem gasdicht geschlossenen System einen kontinuierlichen Strom der freies und/oder freisetzbares Cyanid enthaltenden Probelösung mit den zur Farbbildung, Cyanidfreisetzung und pH-Einstellung erforderlichen Chemikalien unter Einhaltung konstanter Konzentrationen in Kontakt bringt, das Lösungsgemisch zur Bildung des Farbkomplexes in einem rohrförmigen Durchflußreaktor 1 bis 60 Minuten auf 50 bis 120 °C erwärmt, anschließend die Lösung auf die für die Extinktionsmessung vorgesehene Temperatur bringt, den für die Durchflußmeßküvette zulässigen Druck einstellt und dann in an sich bekannter Weise kontinuierlich die Extinktion der durch die Durchflußmeßküvette eines Spektralphotometers strömenden Lösung unter Berücksichtigung der Extinktion einer cyanidfreien Blindprobe mißt.

2. Verfahren nach Anspruch 1, dadurch gekennzeichnet, daß man die zur Farbbildung, Cyanidfreisetzung und pH-Einstellung erforderlichen Chemikalien in Form einer oder mehrerer wäßriger Lösungen der Cyanid enthaltenden Lösung kontinuierlich zusetzt.

3. Verfahren nach Anspruch 1 oder 2, dadurch gekennzeichnet, daß man das Lösungsgemisch im rohrförmigen Durchflußreaktor 5 bis 20 Minuten auf 80 bis 110 °C erwärmt, wobei man im unteren Temperaturbereich bevorzugt länger erwärmt als im oberen Temperaturbereich.

4. Verfahren nach den Ansprüchen 1, 2 oder 3, dadurch gekennzeichnet, daß man Cyanid aus Cyanokomplexen in Gegenwart von vier- oder bevorzugt höherzähligen Chelatkomplexbildnern freisetzt.

5. Verfahren nach Anspruch 4, dadurch gekennzeichnet, daß man Ethylendiamin-tetraessigsäure (EDTA) oder Diethylentriamin-pentaessigsäure (DTPA) oder deren wasserlösliche Salze verwendet.

6. Verfahren nach einem der Ansprüche 1 bis 5, dadurch gekennzeichnet, daß man die Umsetzung zum Farbkomplex bei pH-Werten im Bereich von etwa 7,1 bis etwa 12 vornimmt.

7. Verfahren nach Anspruch 6, dadurch gekennzeichnet, daß man die Umsetzung zum Farbkomplex in Gegenwart von den pH-Wert puffernden Säure-Base-Paaren vornimmt.

8. Verfahren nach den Ansprüchen 1 bis 7, dadurch gekennzeichnet, daß je Liter des pH 8,5 bis 9,5 aufweisenden Lösungsgemischs etwa 0,001 bis 3 mg freies und/oder freisetzbares Cyanid enthalten sind, wobei man zu seiner Herstellung je Liter 0,5 bis 3 g Pikrinsäure, 2 bis 20 g EDTA oder DTPA, 1 bis 10 g NaOH und 1 bis 10 g $Na_2B_4O_7$ verwendet.

9. Verfahren nach den Ansprüchen 1 bis 8, dadurch gekennzeichnet, daß man das Lösungsgemisch nach Verlassen des Durchflußreaktors in einem Durchflußkühler auf 15 bis 30 °C abkühlt und mittels einer Druckregulierungsvorrichtung, vorzugsweise eines Drosselventils, entspannt.

**Claims**

1. A process for the colorimetric determination of the cyanide concentration of aqueous solutions by reaction of the free cyanide and the cyanide releasable from cyano complexes in the presence of chelating agents with picric acid in aqueous alkaline medium at a temperature above room temperature and spectral-photometric measurement of the extinction of the red cyanide/picrate dye complex formed at approximately 520 nm, characterized in that cyanide concentrations of about 0.001 to 5 mg cyanide per litre sample solution are measured by carrying out the determination continuously, for which purpose a continuous stream of the sample solution containing free and/or releasable cyanide is contacted with the chemicals required for dye formation, cyanide liberation and pH adjustment in a gastight system in which constant concentrations are maintained, the solution mixture for forming the dye complex is heated for 1 to 60 minutes at 50 to 120 °C in a tubular throughflow reactor, the solution is subsequently brought to the temperature intended for the extinction measurement, the permitted pressure for the throughflow measuring cell is adjusted and the extinction of the solution flowing through the throughflow measuring cell of a spectral photometer is then continuously measured in known manner taking into account the extinction of a cyanide-free blank sample.

2. A process as claimed in claim 1, characterized in that the chemicals required for dye formation, cyanide liberation and pH adjustment are continuously added to the cyanide-containing solution in the form of one or more aqueous solutions.

3. A process as claimed in claim 1 or 2, characterized in that the solution mixture is heated for 5 to 20 minutes to 80 to 110°C in the tubular throughflow reactor, heating being continued for a longer period in the lower temperature range than in the upper temperature range.

4. A process as claimed in claim 1, 2 or 3, characterized in that cyanide is released from cyano complexes in the presence of fourfold or preferably higher chelating agents.

5. A process as claimed in claim 4, characterized in that ethylenediamine tetraacetic acid (EDTA) or diethylenetriamine pentaacetic acid (DTPA) or water-soluble salts thereof are used.

6. A process as claimed in any of claims 1 to 5, characterized in that the reaction to form the dye complex is carried out at pH values of from about 7.1 to about 12.

7. A process as claimed in claim 6, characterized in that the reaction to form the dye complex is carried out in the presence of pH-buffering acid-base pairs.

8. A process as claimed in claims 1 to 7, characterized in that approximately 0.001 to 3 mg free and/or releasable cyanide are present per litre of the solution mixture having a pH of 8.5 to 9.5, 0.5 to 3 g picric acid, 2 to 20 g EDTA or DTPA, 1 to 10 g NaOH and 1 to 10 g $Na_2B_4O_7$ per litre being used for its production.

9. A process as claimed in claims 1 to 8, characterized in that, after leaving the througflow reactor, the solution mixture is cooled to 15 to 30°C in a throughflow cooler and is expanded by means of a pressure-regulating device, preferably a throttle valve.

**Revendications**

1. Procédé de détermination colorimétrique de la concentration en cyanure de solutions aqueuses par mise en réaction de cyanure libre et de cyanure libérable à partir de complexes cyano en présence d'agents complexants chélatants, avec l'acide picrique en milieu aqueux alcalin à température plus élevée par rapport à la température ordinaire, puis mesure spectrophotométrique de l'extinction du complexe coloré rouge cyanure/picrate à environ 520 nm, caractérisé en ce que l'on effectue le dosage à des concentrations de cyanures dans la zone d'environ 0,001 à 5 mg par litre de solution d'essai en effectuant la détermination en continu, procédé dans lequel on met en contact dans un système clos étanche aux gaz une solution d'essai contenant le cyanure libre ou libérable, avec les produits chimiques nécessaires pour la formation de la coloration, la libération du cyanure et/ou l'ajustement du pH tout en maintenant des concentrations constantes, on chauffe le mélange de solutions jusqu'à la formation du complexe coloré dans un réacteur à écoulement de forme tubulaire pendant 1 à 60 mn de 50 à 120°C, ensuite on porte la solution à la température prévue pour la mesure de l'extinction, on ajuste la pression compatible pour la cuvette de mesure par écoulement et ensuite on mesure d'une façon connue en soi en continu l'extinction de la solution qui passe à travers la cuvette de mesure par écoulement tout en tenant compte de l'extinction d'un essai à blanc dépourvu de cyanure.

2. Procédé selon la revendication 1, caractérisé en ce que l'on ajoute les produits chimiques nécessaires pour la formation de la couleur, la libération du cyanure et l'ajustement du pH sous la forme d'une ou plusieurs solutions aqueuses de solution contenant le cyanure, d'une manière continue.

3. Procédé selon la revendication 1 ou 2, caractérisé en ce que l'on chauffe le mélange de solutions dans un réacteur à écoulement de forme tubulaire pendant 5 à 20 mn à 80-110°C, chauffage dans lequel on chauffe plus longtemps dans la zone inférieure de température que dans la zone supérieure de température.

4. Procédé selon les revendications 1, 2 ou 3, caractérisé en ce qu'on libère le cyanure des complexes cyano en présence d'agents complexants chélatants à quatre chiffres ou de préférence à chiffres plus élevés.

5. Procédé selon la revendication 4, caractérisé en ce que l'on utilise l'acide éthylène diamine tétraacétique (EDTA) ou l'acide diéthylènetriaminopentaacétique (DTPA) ou leurs sels solubles dans l'eau.

6. Procédé selon l'une des revendications 1 à 5, caractérisé en ce que l'on effectue la réaction en vue du complexe coloré à des valeurs de pH dans la zone d'environ 7,1 à environ 12.

7. Procédé selon la revendication 6, caractérisé en ce que l'on effectue la réaction en vue du complexe coloré en présence de paires acide/base qui servent à tamponner la valeur de pH.

8. Procédé selon les revendications 1 à 7, caractérisé en ce que par litre du mélange de solution possédant un pH de 8,5 à 9,5 environ 0,001 à 3 mg de cyanure libre ou libérable sont contenus, pour lesquels on utilise pour leur production par litre 0,5 à 3 g d'acide picrique, 2 à 20 g d'EDTA ou DTPA, 1 à 10 g de NaOH et 1 à 10 g de $B_4O_7Na_2$.

9. Procédé selon les revendications 1 à 8, caractérisé en ce que l'on refroidit le mélange de solutions après sa sortie du réacteur par écoulement dans un réfrigérant par écoulement à 15-30° C, et que l'on détend à l'aide d'un dispositif de réglage de pression, de préférence une soupape à étranglement.